# EUROPEAN PATENT APPLICATION

(11) **EP 4 755 386 A1**
(43) Date of publication of application: **10.06.2026**
(21) Application number: 24848816.5
(22) Date of filing: 05.07.2024
(51) Int. Cl.: A61K 31/7048, A61K 31/196, A61K 31/352, A61P 29/00, A61P 43/00

(54) **SKIN PERMEATION PROMOTER AND SKIN PERMEATION PROMOTION METHOD**

(30) Priority: 28.07.2023 JP 2023123149
(71) Applicant: Toyo Sugar Refining Co., Ltd., Tokyo 1030006 (JP)
(72) Inventor: TOZUKA Yuichi, Takatsuki-shi, Osaka 569-1094 (JP); KADOTA Kazunori, Takatsuki-shi, Osaka 569-1094 (JP); UCHIYAMA Hiromasa, Takatsuki-shi, Osaka 569-1094 (JP); NAKANISHI Akihito, Ichihara-shi, Chiba 290-0046 (JP); TANDIA Mahamadou, Noda-shi, Chiba 278-0027 (JP)
(74) Representative: Dehns
(86) International application number: PCT/JP2024/024376
(87) International publication number: WO 2025/028157

(57) **Abstract**

The present disclosure relates to a skin permeation enhancer for allowing a bioactive substance to permeate, the skin permeation enhancer containing α-glucosylnaringin, and a skin permeation enhancement method.

## Description

### Technical Field

The present disclosure relates to a skin permeation enhancer and a skin permeation enhancement method.

### Background Art

The skin has attracted attention as an administration site of a drug (bioactive substance) that is expected to have not only a local action but also a systemic action, because, for example, the first pass effect in the liver can be avoided, an administration method is simple, and administration interruption is also simple. Since it is necessary for an administered drug to permeate through the skin in order to exhibit the drug efficacy, various attempts have been made to allow the drug to permeate therethrough.

The skin permeation pathway of the drug includes a stratum corneum pathway for permeation through the intercellular space of the stratum corneum or inside the stratum corneum cell, and an appendageal pathway for permeation through an appendage such as a hair follicle or a sweat gland, and the main permeation pathway is a stratum corneum pathway. Since the stratum corneum is a dense membrane having high lipid solubility as a whole and functions as a barrier for protecting the living body from the outside world, many drugs do not permeate through the skin. In particular, a drug having a large molecular weight or a water-soluble drug has remarkably low skin permeability (Non Patent Literature 1).

As a permeation enhancer for a drug, for example, aliphatic alcohols and fatty acids, esters thereof, monoterpenes, and essential oils are known, but since these act on stratum corneum lipids and reduce the barrier ability thereof to improve the skin permeability of a drug (Non Patent Literature 1), there are many cases where there is a problem in safety. In addition, depending on the drug, skin permeability is often insufficient even with the use of a permeation enhancer.

It has been reported that when quercetin, apigenin, or myricetin, each of which is a flavonoid compound, is used as a drug permeation enhancer having low irritation to the skin, skin permeation of a drug such as loxoprofen sodium, a specific peptide, aspirin, imiquimod, or epinephrine is enhanced (Patent Literature 1).

It has been reported that α-glucosylnaringin, which is a type of glucosylated flavonoid compound, can improve the solubility of a specific poorly water-soluble substance in water more than before, thereby improving the absorbability in the body, and can exhibit a desired drug efficacy with a smaller amount used (Patent Literature 2). However, Patent Literature 2 merely shows a prediction of the absorbability of the poorly water-soluble substance in the body, and how α-glucosylnaringin acts on skin permeation of a drug is not known at all.

### Citation List

### Patent Literature

Patent Literature 1: JP 2015-163606 A
Patent Literature 2: JP 2017-48127 A

### Non Patent Literature

Non Patent Literature 1: "Transdermal delivery of mal-absorbable drugs with chemical and physical enhancement methods", Hiroaki Todo, et al. Drug Deliverly System, 27-3, 2012, pp. 156-163

### Summary of Invention

### Technical Problem

The flavonoid compound described in Patent Literature 1, such as quercetin, apigenin, or myricetin, is poorly soluble in water, and thus is difficult to be blended in, for example, an aqueous preparation.

In view of the above circumstances, the present disclosure provides a skin permeation enhancer that allows a bioactive substance to permeate, is easily blended in, for example, an aqueous preparation, and has low irritation to the skin, and a skin permeation enhancement method for a bioactive substance.

### Solution to Problem

The present inventors have intensively studied to solve the above problem. As a result, the present inventors have found that the above problem can be solved by having the following configuration, and have completed the present invention.

The present invention relates to, for example, the following [1] to [7].
[1] A skin permeation enhancer for allowing a bioactive substance to permeate, the skin permeation enhancer containing α-glucosylnaringin.
[2] The skin permeation enhancer according to [1], wherein the α-glucosylnaringin contains α-monoglucosylnaringin.
[3] The skin permeation enhancer according to [2], wherein a content of the α-monoglucosylnaringin is 65 mass% or more in 100 mass% of the skin permeation enhancer.
[4] The skin permeation enhancer according to any one of [1] to [3], wherein the bioactive substance is at least one selected from the group consisting of an acidic nonsteroidal anti-inflammatory drug (NSAID) and a flavonoid.
[5] The skin permeation enhancer according to [4], wherein the acidic nonsteroidal anti-inflammatory drug (NSAID) is an aromatic compound having two or more benzene rings.
[6] The skin permeation enhancer according to any one of [1] to [4], wherein the bioactive substance is at least one selected from the group consisting of mefenamic acid or a salt thereof, flavone, and hesperetin.
[7] A skin permeation enhancement method for a bioactive substance, the method including a step of topically administering α-glucosylnaringin and a bioactive substance to the skin.

### Advantageous Effects of Invention

According to the present disclosure, it is possible to provide a skin permeation enhancer that allows a bioactive substance to permeate, is easily blended in, for example, an aqueous preparation, and has low irritation to the skin, and a skin permeation enhancement method for a bioactive substance.

### Brief Description of Drawings

Fig. 1 is a graph showing a change in skin permeation amount of flavone over time when α-glucosylnaringin, α-glucosylrutin, α-glucosylhesperidin, or naringin was added.
Fig. 2 is a graph showing a change in skin permeation amount of flavone over time when α-glucosylnaringin was added.
Fig. 3 is a graph showing a change in skin permeation amount of hesperetin over time when α-glucosylnaringin was added.
Fig. 4 is a graph showing a change in skin permeation amount of mefenamic acid over time when α-glucosylnaringin was added.

### Description of Embodiments

Next, the present invention will be specifically described.

The expression "A to B" in the numerical range means A or more and B or less unless otherwise specified. In addition, % means mass%.

### <α-Glucosylnaringin>

α-Glucosylnaringin (also referred to as α-glucosylnaringin) is a generic term for compounds in which one or more molecules of glucose are added to the hydroxyl group of naringin. Naringin is a type of flavonoid having a structure in which neohesperidose (L-rhamnosyl-(α1 → 2)-D-glucose) is β-bonded to the hydroxyl group at the 7 position of the naringenin (5,7,4'-trihydroxyflavanone) skeleton. α-Glucosylnaringin has a structure in which one or more molecules of α-glucose are bonded to at least one of the hydroxyl group at the 3 position (3" position) of the glucose residue in the neohesperidose residue and the hydroxyl group at the 4 position (4' position) of the phenyl group in the naringenin skeleton of the naringin. The α-glucosylnaringin in the present invention may be formed of only one type of the compounds having such a structure, or may be a mixture of two or more types thereof.

α-Glucosylnaringin can be represented by the following formula (1). In the formula (1), m of R¹ and n of R² mean the number of α-glucose residues bonded to the hydroxyl group at the 3" position and the hydroxyl group at the 4' position, respectively, and each independently represent an integer of 0 or more and usually 25 or less. However, in order for the formula (1) to represent "α-glucosylnaringin", it is necessary to satisfy m + n ≥ 1, that is, at least one molecule of α-glucose needs to be linked to naringin (when m = n = 0, that is, when both R¹ and R² are -H, the formula (1) represents "naringin").

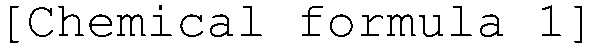

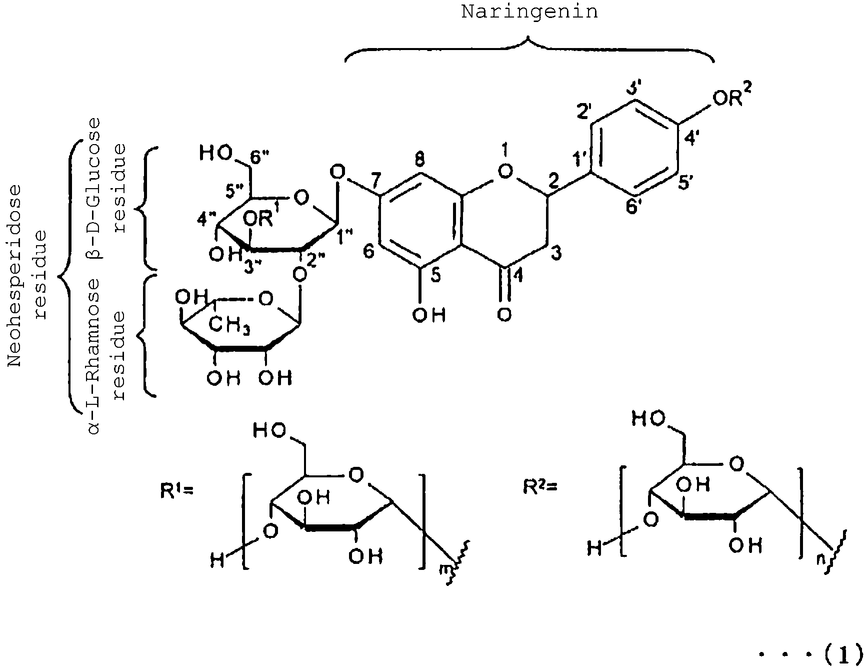

α-Glucosylnaringin is a compound contained as a main component in a material known as "enzyme-treated naringin" (sometimes also referred to as "transglycosylated naringin"). Among α-glucosylnaringin, one to which only one glucose molecule is bound is referred to as "α-monoglucosylnaringin", and one to which two or more glucose molecules are bound is referred to as "α-polyglucosylnaringin".

The enzyme-treated naringin is, for example, a product (herein, referred to as "first enzyme-treated naringin") obtained by reacting a mixture of naringin and a sugar donor (for example, dextrin) with a glycosyltransferase (for example, cyclodextrin glucosyltransferase), and is an aggregate of various compounds in which one or more molecules of glucose are added to the hydroxyl group of naringin. Accordingly, the enzyme-treated naringin usually includes a mixture of compounds that differ in the number of glucose molecules bound to the naringin, such as a mixture containing α-monoglucosylnaringin and α-polyglucosylnaringin. In addition to α-glucosylnaringin, unreacted naringin or naringin derivatives other than α-glucosylnaringin such as 7-glucosylnaringenin (also referred to as other naringin derivatives) may be contained.

For a basic method for producing the first enzyme-treated naringin, for example, JP H04-13691 A can be referred to. If necessary, the first enzyme-treated naringin with the purity of α-glucosylnaringin increased (α-glucosylnaringin purified product) is obtained by purifying the first enzyme-treated naringin using, for example, a porous synthetic adsorbent and an appropriate eluate to remove the sugar donor and other impurities, and further reduce the content of naringin.

As the α-glucosylnaringin, at least one α-glucosylnaringin selected from those in which one molecule of α-glucose is linked to the hydroxyl group at the 3" position of naringin and/or one molecule of α-glucose is linked to the hydroxyl group at the 4' position of naringin, that is, 3"-α-monoglucosylnaringin (in the formula (1), m = 1, n = 0), 4'-α-monoglucosylnaringin (in the formula (1), m = 0, n = 1), and 3"-4'-α-diglucosylnaringin (in the formula (1), m = 1, n = 1) is preferable, and among them, 3"-α-monoglucosylnaringin is more preferable. This is because since the molecular weight of α-monoglucosylnaringin is smaller than the molecular weight of α-polyglucosylnaringin, the number of molecules of α-monoglucosylnaringin per unit mass is larger, which is considered advantageous in terms of skin permeation enhancement effect.

The α-glucosylnaringin preferably contains α-monoglucosylnaringin.

The enzyme-treated naringin containing a large amount of the three types of α-mono/diglucosylnaringin (herein, referred to as "second enzyme-treated naringin") can be obtained, for example, by treating the above-described first enzyme-treated naringin with an enzyme having glucoamylase activity, and cleaving a sugar chain in which two or more molecules of α-glucose are bonded to each other by an α-1,4 linkage, the sugar chain having been transferred to the hydroxyl group at the 3" position and/or the hydroxyl group at the 4' position of naringin by the glycosyltransferase, while leaving only an α-glucose residue corresponding to one molecule at the base. Furthermore, the second enzyme-treated naringin is treated with an enzyme having α-glucosidase activity, and the α-glucose residue corresponding to one molecule that is directly bonded to the hydroxyl group at the 4' position is cleaved, whereby an enzyme-treated naringin leaving 3"-α-monoglucosylnaringin and containing little or no 4'-α-monoglucosylnaringin and 3"-4'-α-diglucosylnaringin (herein, referred to as "third enzyme-treated naringin") can be obtained. For a basic method for producing the second and third enzyme-treated naringins, for example, JP 2002-199896 A can be referred to.

Furthermore, 7-glucosylnaringenin is produced by allowing α-L-rhamnosidase to act on the third enzyme-treated naringin to cleave rhamnose contained in the rutinose unit of the naringin, whereby an enzyme-treated naringin containing 7-glucosylnaringenin and α-monoglucosylnaringin (herein, referred to as "fourth enzyme-treated naringin") is obtained.

In addition, when the transglucosidase is allowed to act on the first enzyme-treated naringin, since the transglucosidase is an enzyme having both glucoamylase activity and α-glucosidase activity, the third enzyme-treated naringin rich in 3"-α-monoglucosylnaringin is obtained by a one-step treatment instead of the two-step treatment as described above. Furthermore, if necessary, a treatment may be performed in which an enzyme having β-glucosidase activity is allowed to act on the third enzyme-treated naringin (the enzyme may be allowed to act simultaneously with the transglucosidase on the first enzyme-treated naringin) to cleave the neohesperidose residue of unreacted naringin (whose sugar chain has not been modified by glycosyltransferase) dissolved in a small amount in an aqueous solution from naringenin that is an aglycone of the neohesperidose residue. Since naringenin produced by such a treatment has lower solubility than that of naringin, naringenin can be easily removed from the aqueous solution through precipitation, so that 3"-α-monoglucosylnaringin can be collected with higher purity from the aqueous solution.

In the present invention, in consideration of, for example, the effect of the present invention, an enzyme-treated naringin which is a composition containing α-glucosylnaringin is preferably used, or any composition of first enzyme-treated naringin, second enzyme-treated naringin, third enzyme-treated naringin, and fourth enzyme-treated naringin may be used, but preferably fourth enzyme-treated naringin is used.

In consideration of, for example, the effect of the present invention, the enzyme-treated naringin is preferably a mixture containing α-glucosylnaringin and at least one selected from the group consisting of naringin and 7-glucosylnaringenin.

The presence of various α-glucosylnaringin, naringin, and other components contained in the enzyme-treated naringin can be confirmed by a chromatogram of HPLC, and the content of each component or the purity of a desired specific component can be calculated from the peak area of the chromatogram.

The method for producing α-glucosylnaringin is not particularly limited, and a known method can be used. From the viewpoint of good yield and easy production, the enzyme treatment of naringin described above is preferable for production. The method for obtaining and preparing naringin is not particularly limited, and a compound generally produced and sold as a reagent or a purified product may be used, or a compound prepared by extraction from raw materials such as citrus (such as Natsumikan and grapefruit) peel may be used.

### <Bioactive substance>

The bioactive substance is not particularly limited as long as it is a substance known to have bioactivity.

Examples of the bioactive substance include an anti-inflammatory drug, an antibacterial agent, a hormone agent, an antihistamine, an antiallergic agent, a blood flow improving agent, a vasodilator, a cardiotonic agent, an antiarrhythmic agent, an antianginal agent, an antihypertensive agent, an insulin preparation, a sequestering agent, vitamins, an emollient, a humectant, a whitening agent, an antiwrinkle agent, an astringent agent, an ultraviolet absorber, a hair growth agent, a hair restorer, an antiperspirant, and a deodorant, for example.

The bioactive substance is preferably a poorly water-soluble substance, more preferably a poorly water-soluble substance having two or more benzene rings because the skin permeation enhancement effect of α-glucosylnaringin is easily exhibited.

More specific examples of the anti-inflammatory drug include a steroidal anti-inflammatory drug and a nonsteroidal anti-inflammatory drug (NSAID), and the anti-inflammatory drug is preferably a nonsteroidal anti-inflammatory drug, and more preferably an acidic nonsteroidal anti-inflammatory drug (NSAID) .

Examples of the acidic nonsteroidal anti-inflammatory drug (NSAID) include salicylic acid-based ones such as aspirin, ethenzamide, diflusinal (diflunisal), and methyl salicylate; propionic acid-based ones such as ibuprofen, naproxen, fenoprofen, ketoprofen, dexketoprofen, flurbiprofen, oxaprozin, and loxoprofen; acetic acid-based ones such as indomethacin, tolmetin, sulindac, etodolac, ketorolac, and diclofenac; oxicam-based ones such as piroxicam, meloxicam, tenoxicam, lornoxicam, and isoxicam; phenamic acid-based ones such as mefenamic acid, meclofenamic acid, flufenamic acid, and tolfenamic acid; and salts thereof.

The salt may be a pharmaceutically acceptable salt, and examples thereof include inorganic salts containing a metal such as sodium, potassium, magnesium, calcium, aluminum, or lithium, organic salts such as, methylamine, ethylamine, ethanolamine, or lysine, phosphate salts, and ammonium salts, for example.

Among the acidic nonsteroidal anti-inflammatory drugs (NSAIDs), an aromatic compound having two or more benzene rings is preferable because the skin permeation enhancement effect of α-glucosylnaringin is easily exhibited. Examples of the aromatic compound having two or more benzene rings include diflusinal (diflunisal), naproxen, fenoprofen, ketoprofen, dexketoprofen, flurbiprofen, oxaprozin, indomethacin, sulindac, diclofenac, piroxicam, mefenamic acid, meclofenamic acid, flufenamic acid, and tolfenamic acid, and fenamic acid-based ones such as mefenamic acid, meclofenamic acid, flufenamic acid, and tolfenamic acid are preferable.

More specific examples of the blood flow improving agent include flavonoids, tocopherol or derivatives thereof, heparin analogues, dl-camphor, ginkgo leaf extract, ginger rhizome extract, capsicum fruit extract, ginger extract, cilostazol, ticlopidine hydrochloride, alprostadil, limaprost, beraprost sodium, sarpogrelate hydrochloride, argatroban, naftidrofuryl, isoxsuprine hydrochloride, batroxobin, dihydroergotoxine mesylate, tolazoline hydrochloride, hepronicart, and Si-Wu-Tang extract. Among them, flavonoids are preferable because the skin permeation enhancement effect of α-glucosylnaringin is easily exhibited.

Examples of the flavonoids include flavones such as flavone, apigenin, luteolin, tangeretin, diosmin, nobiletin, and flavoxate; isoflavones such as daidzein, daidzin, and genistein; flavonols such as kaempferol, myricetin, quercetin, and rutin; flavanones such as eriodictyol, hesperetin, hesperidin, homoeriodictyol, and naringenin; flavanols such as catechin, epicatechin, and epigallocatechin; flavanonols such as dihydroquercetin; and anthocyanidins such as cyanidin, anthocyanin, delphinidin, malvidin, pelargonidine, and peonidin, for example.

Among them, flavonols, flavones, and flavanones are preferable, rutin, quercetin, hesperidin, flavone, and hesperetin are more preferable, and flavone and hesperetin are still more preferable because the skin permeation enhancement effect of α-glucosylnaringin is easily exhibited.

The bioactive substance is preferably at least one selected from the group consisting of acidic nonsteroidal anti-inflammatory drugs (NSAIDs) and flavonoids because the skin permeation enhancement effect of α-glucosylnaringin is easily exhibited.

The bioactive substance is preferably at least one selected from the group consisting of mefenamic acid or a salt thereof, flavone, and hesperetin, because the skin permeation enhancement effect of α-glucosylnaringin is easily exhibited.

### <Skin permeation enhancer>

A skin permeation enhancer according to an aspect of the present invention contains α-glucosylnaringin, and enhances the skin permeation of a bioactive substance.

The method for evaluating the effect of enhancing skin permeation is not particularly limited, and a known method can be used. For example, when the degree of skin permeation of a bioactive substance when a test substance is added is higher than the degree of skin permeation of the bioactive substance when the test substance is not added, it can be evaluated that the test substance has an effect of enhancing skin permeation.

The degree of skin permeation of a bioactive substance may be evaluated at one time point, or may be evaluated at a plurality of different time points, that is, over time. The time point at which the degree of skin permeation of a bioactive substance is evaluated is not particularly limited, but is preferably 4 hours or more after the start of the test, more preferably 6 hours or more after the start of the test, still more preferably 8 hours or more after the start of the test, and particularly preferably 12 hours or more after the start of the test.

The degree of skin permeation of a bioactive substance can be measured by a known method, but is preferably measured by a method using a Franz-type diffusion cell. As a permeable membrane of a Franz-type diffusion cell, a known membrane, for example, a skin derived from an animal such as a human being, a guinea pig, or a mouse, a three-dimensional cultured skin, or an artificial membrane for a skin permeability test such as PermeaPad Barrier or Strat-M membrane can be used.

The degree of enhancing skin permeation is not particularly limited as long as the skin permeability of a bioactive substance in the case of using α-glucosylnaringin is greater than 100% when the skin permeability of a bioactive substance in the case of not using α-glucosylnaringin (in the case of using the bioactive substance alone) is taken as 100%, but is, for example, 110% or more, 120% or more, 130% or more, 140% or more, 150% or more, 200% or more, 250% or more, 300% or more, 350% or more, 400% or more, 450% or more, or 500% or more.

The amount of α-glucosylnaringin contained in the skin permeation enhancer is not particularly limited. For example, the lower limit of the amount of α-glucosylnaringin contained in the skin permeation enhancer of the present invention is, for example, 30 mass%, 40 mass%, 45 mass%, 50 mass%, 60 mass%, 70 mass%, or 80 mass%. The upper limit of the amount of α-glucosylnaringin contained in the skin permeation enhancer of the present invention is, for example, 100 mass%, 99 mass%, 98 mass%, 95 mass%, 90 mass%, or 85 mass%. As the range of the amount of α-glucosylnaringin contained in the skin permeation enhancer of the present invention, a range in which the lower limit and the upper limit are arbitrarily combined can be arbitrarily set, and for example, a range of 30 to 100 mass%, 60 to 100 mass%, or 60 to 90 mass%, for example, can be set. The content of α-glucosylnaringin is preferably 65 mass% or more, more preferably 70 mass% or more in 100 mass% of the skin permeation enhancer, and further preferably the content of α-monoglucosylnaringin is 65 mass% or more, particularly preferably 70 mass% or more in 100 mass% of the skin permeation enhancer.

The skin permeation enhancer need only contain α-glucosylnaringin, and may be formed only of α-glucosylnaringin, or may further contain a known optional component such as an excipient, a stabilizer, a wetting agent, or an emulsifier as long as the skin permeation enhancement effect of α-glucosylnaringin is not hindered. The skin permeation enhancer may also be enzyme-treated naringin, which is a composition containing α-glucosylnaringin.

The mechanism by which α-glucosylnaringin enhances the skin permeation of a bioactive substance is not clear, but the present inventors presume as follows.

Since the skin is formed of lipids, it is generally known that the permeability of a water-soluble drug to the skin is remarkably low, and the higher the lipid solubility, the easier the distribution into the skin and the higher the skin permeation rate. That is, it is a technical common knowledge that it is considered that the skin permeability decreases when a poorly water-soluble substance is solubilized to increase the water solubility.

However, the present inventors believe that two factors, solubility and subsequent releasability, greatly contribute to the skin permeation of a poorly water-soluble substance. That is, in order to enhance the skin permeation of a poorly water-soluble substance, first, it is necessary to solubilize the poorly water-soluble substance to increase the water solubility, and second, it is necessary that the poorly water-soluble substance after being solubilized is sufficiently released to the skin.

It has been reported that α-glucosylnaringin can improve the solubility of a predetermined poorly water-soluble substance in water more than before (Patent Literature 2). Since α-glucosylnaringin solubilizes a poorly water-soluble substance to increase water solubility, it is predicted based on common technical knowledge that when α-glucosylnaringin is added, the skin permeability of the poorly water-soluble substance decreases. However, as shown in examples herein, α-glucosylnaringin remarkably enhanced the skin permeation of a poorly water-soluble substance as compared with naringin having no solubilizing effect.

In addition, it has been reported that not only α-glucosylnaringin but also α-glucosylhesperidin and α-glucosylrutin, which are glucosylated flavonoid compounds, can improve the solubility of a poorly water-soluble substance in water more than before (Patent Literature 2). However, as shown in examples herein, only α-glucosylnaringin remarkably enhanced the skin permeation of a bioactive substance, and the skin permeation enhancement effect of α-glucosylhesperidin or α-glucosylrutin was slight.

From these facts, it is considered that α-glucosylnaringin has a higher action of enhancing the releasability of a poorly water-soluble substance after being solubilized than α-glucosylhesperidin or α-glucosylrutin, and the high skin permeation enhancement effect of α-glucosylnaringin is mainly due to this action.

The use of the skin permeation enhancer is not particularly limited, but the skin permeation enhancer of the present invention has a high action of enhancing the skin permeation of a bioactive substance, and therefore, for example, can be administered to a living body in a single dose or multiple doses for the purpose of enhancing the skin permeation of a bioactive substance in the living body.

The skin permeation enhancer administered to a living body enhances the skin permeation of a bioactive substance, and thus contributes to, for example, the expression of the activity of the bioactive substance locally or systemically.

### <Administration of skin permeation enhancer to living body>

When the skin permeation enhancer is administered to a living body, the administration route is usually topical application to the skin.

The method for administering the skin permeation enhancer is not particularly limited, and the skin permeation enhancer and a bioactive substance whose permeation is to be enhanced may be administered in combination, and usually, the skin permeation enhancer and the bioactive substance whose permeation is to be enhanced are administered simultaneously.

The skin permeation enhancer may be administered to a living body as it is, but is preferably administered as a preparation in which an effective amount of the skin permeation enhancer and a bioactive substance whose permeation is to be enhanced are combined.

The content of the skin permeation enhancer in the preparation may be appropriately selected depending on the type or concentration of the bioactive substance whose permeation is to be enhanced. From the viewpoint of the skin permeation enhancement effect, the preparation preferably contains 0.01 to 5 mass%, more preferably 0.025 to 2 mass%, and still more preferably 0.05 to 1 mass% of α-glucosylnaringin in 100 mass% of the preparation.

The concentration of the bioactive substance in the preparation may be appropriately selected depending on, for example, the type of the bioactive substance, the site (local or systemic) where the activity is expressed, or the degree of the activity desired to be expressed. When the bioactive substance is flavone, hesperetin, or mefenamic acid, the concentration of the bioactive substance in the preparation is preferably 0.01 to 5 mg/mL, and more preferably 0.1 to 1 mg/mL.

The ratio of α-glucosylnaringin to the bioactive substance in the preparation may be appropriately selected depending on, for example, the type of the bioactive substance, the site (local or systemic) where the activity is expressed, or the degree of the activity desired to be expressed. The mass ratio of α-glucosylnaringin to the bioactive substance in the preparation is preferably 150:1 to 1:1, and more preferably 40:1 to 2:1. When the bioactive substance is flavone, hesperetin, or mefenamic acid, the mass ratio of α-glucosylnaringin to the bioactive substance in the preparation is preferably 100:1 to 2:1, and more preferably 40:1 to 4:1.

The preparation may be any of a pharmaceutical product, a quasi drug, and a cosmetic as long as it is a preparation that can be applied to the skin.

The properties and dosage form of the preparation are not limited, and the preparation may be, for example, in a solid form, a semi-solid form, a liquid form, an emulsion form, a cream form, a gel form, a mousse form, a spraying agent, or a two-pack form to be used by mixing these, a liniment, a cataplasm, a plaster, a patch (example: a film, a tape), for example.

In the preparation, components usually used in, cosmetics, topical agents for skin such as pharmaceuticals, or cleaning agents, for example, fats and oils, waxes, hydrocarbon oils, ester oils, higher alcohols, silicone oils, ultraviolet absorbers, ultraviolet scattering agents, humectants, surfactants, water-soluble polymers, thickeners, powders, skin protecting agents, skin lightening agents, wrinkle improving agents, antiaging agents, plant extracts, preservatives, anti-inflammatory agents, pH adjusting agents, sequestering agents, antioxidants, stabilizers, fragrances, dyes, and pigments, for example, can be appropriately blended as necessary as long as the effects of the present invention are not impaired. These components may be used alone or in combination of two or more thereof.

The preparation can be produced by adding a skin permeation enhancer and a bioactive substance according to a method generally used for such a preparation. The skin permeation enhancer and the bioactive substance may be added at the initial stage of the production step of the preparation, or may be added at the middle or final stage of the production step, and the addition method may be appropriately selected from, for example, mixing, kneading, dissolution, immersion, dispersion, spraying, and application depending on the aspect of the preparation.

Since α-glucosylnaringin, which is an active ingredient of the skin permeation enhancer, has good water solubility, the skin permeation enhancer of the present invention has high water solubility, can be uniformly dissolved or dispersed even when added to water or a preparation containing a large amount of water, and is easily blended, for example, in an aqueous preparation. Therefore, the skin permeation enhancer of the present invention can be blended in a very wide range of preparations.

α-Glucosylnaringin, which is an active ingredient of the skin permeation enhancer, has low irritation to the skin, is rich in experience of eating, and has good safety, and therefore, the skin permeation enhancer of the present invention has low irritation to the skin and high safety.

### <Skin permeation enhancement method>

One aspect of the present invention is a skin permeation enhancement method for a bioactive substance, the method including a step of topically administering α-glucosylnaringin and a bioactive substance to the skin.

The method for topically administering α-glucosylnaringin and a bioactive substance to the skin is not particularly limited, and is as described in the section of <Administration of skin permeation enhancer to living body>.

One aspect of the present invention is the use of α-glucosylnaringin for enhancing the skin permeation of a bioactive substance.

One aspect of the present invention is the use of α-glucosylnaringin in the production of a skin permeation enhancer for permeation of a bioactive substance.

### Examples

Next, the present invention will be described in more detail with reference to examples, but the present invention is not limited thereto.

### <Skin permeability test 1 using Franz-type diffusion cell>

### (Method)

As the substance group A, the following α-glucosylnaringin (Example 1), naringin (Comparative Example 1), α-glucosylhesperidin (Comparative Example 2), and α-glucosylrutin (Comparative Example 3) were used.
α-Glucosylnaringin (Naringin-G): enzyme-treated naringin (manufactured by Toyo Sugar Refining Co., Ltd., 75 mass% of α-monoglucosylnaringin and trace amounts of naringin and 7-glucosylnaringenin are contained in 100 mass% of enzyme-treated naringin)
Naringin: naringin reagent (manufactured by Tokyo Chemical Industry Co., Ltd., 96 mass% of naringin is contained in 100 mass% of naringin reagent)
α-Glucosylhesperidin (Hsp-G): enzyme-treated hesperidin (manufactured by Toyo Sugar Refining Co., Ltd., 80 mass% of α-monoglucosylhesperidin is contained in 100 mass% of enzyme-treated hesperidin)
α-Glucosylrutin (Rutin-G): enzyme-treated rutin (manufactured by Toyo Sugar Refining Co., Ltd., 75 mass% of α-monoglucosylrutin is contained in 100 mass% of enzyme-treated rutin)

As a bioactive substance, flavone (manufactured by Tokyo Chemical Industry Co., Ltd., NO. H0721) was used.

Any substance in the substance group A and a bioactive substance were added to 10 mL of distilled water and mixed to prepare a solution (or suspension), which was used as a test liquid. The substance in the substance group A was added in an amount such that the concentration in the test liquid was 10 mg/mL. The bioactive substance was added in an amount such that the concentration in the test liquid was 250 µg/mL. As a control, a test liquid (flavone only) to which a substance in the substance group A was not added was also prepared.

A receptor part of a Franz-type vertical diffusion cell was filled with a phosphate buffer (pH 7.4) containing 0.5% sodium taurocholate, and a Strat-M membrane for a transdermal diffusion test (manufactured by Merck) was attached as a permeable membrane. After 400 µL of the test liquid was dropped on the donor side, a receptor liquid was sampled over time. The amount of the bioactive substance in the sampled receptor liquid was quantified by high performance liquid chromatography. The experiment was performed with N = 1.

### (Results)

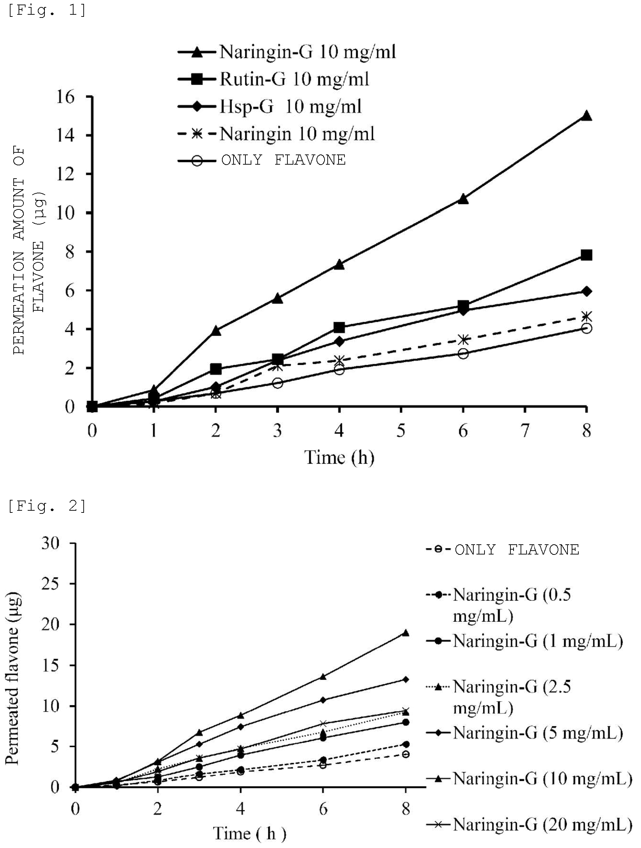

The results are shown in Fig. 1.

When naringin which is a poorly water-soluble flavonoid compound was added, the skin permeation amount of flavone hardly changed. When α-glucosylhesperidin or α-glucosylrutin, each of which is a water-soluble glucosylated flavonoid compound, was added, the skin permeation amount of flavone increased, but the magnitude of the increase was small.

On the other hand, when α-glucosylnaringin was added, the skin permeation amount of flavone significantly increased. That is, it was revealed that α-glucosylnaringin enhances the skin permeation of a bioactive substance.

### <Skin permeability test 2 using Franz-type diffusion cell >

### (Method)

As a substance in the substance group A, α-glucosylnaringin (Naringin-G), which was the same as in the skin permeability test 1, was used.

As the bioactive substance, flavone (manufactured by Tokyo Chemical Industry Co., Ltd., NO. H0721, Example 2), hesperetin (manufactured by Sigma-Aldrich Co. Ltd., NO. F2003, Example 3), and mefenamic acid (manufactured by Tokyo Chemical Industry Co., Ltd., NO. M1782, Example 4) were used.

α-Glucosylnaringin and any of the bioactive substances were added to 10 mL of distilled water and mixed to prepare a solution (or suspension), which was used as a test liquid. α-Glucosylnaringin was added at a concentration of 0.5 mg/mL, 1 mg/mL, 2.5 mg/mL, 5 mg/mL, 10 mg/mL, or 20 mg/mL(an amount to give 10 mg/mL or 25 mg/mL with respect to mefenamic acid and an amount to give 1 mg/mL or 20 mg/mL with respect to hesperetin) in the test liquid. The bioactive substance was added in an amount such that the concentration in the test liquid was 250 µg/mL. As controls, test liquids (flavone only, hesperetin only, mefenamic acid only) to which a substance in the substance group A was not added were also prepared.

A skin permeability test using a Franz-type diffusion cell was performed in the same manner as in the skin permeability test 1. The experiment was performed with N = 3, and the average value was calculated.

### (Results)

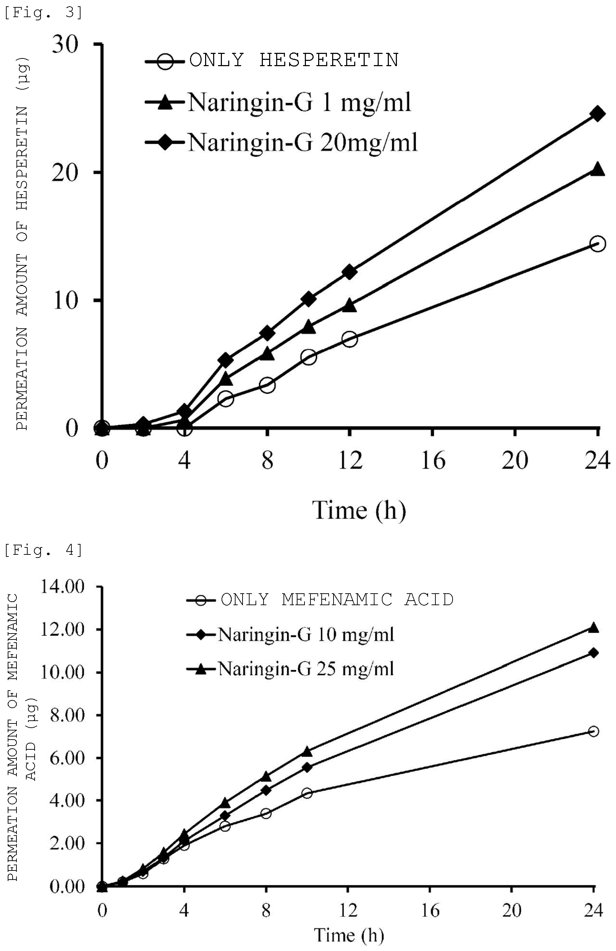

Fig. 2 shows the results when flavone was used as the bioactive substance (Example 2), Fig. 3 shows the results when hesperetin was used as the bioactive substance (Example 3), and Fig. 4 shows the results when mefenamic acid was used as the bioactive substance (Example 4).

From Figs. 2, 3, and 4, the skin permeation amounts of flavone, hesperetin, and mefenamic acid increased by the addition of α-glucosylnaringin. The skin permeation enhancing action of α-glucosylnaringin was dependent on the concentration of α-glucosylnaringin within a given concentration range.

## Claims

1. A skin permeation enhancer for allowing a bioactive substance to permeate, the skin permeation enhancer comprising α-glucosylnaringin.

2. The skin permeation enhancer according to claim 1, wherein the α-glucosylnaringin contains α-monoglucosylnaringin.

3. The skin permeation enhancer according to claim 2, wherein a content of the α-monoglucosylnaringin is 65 mass% or more in 100 mass% of the skin permeation enhancer.

4. The skin permeation enhancer according to claim 1, wherein the bioactive substance is at least one selected from the group consisting of an acidic nonsteroidal anti-inflammatory drug (NSAID) and a flavonoid.

5. The skin permeation enhancer according to claim 4, wherein the acidic nonsteroidal anti-inflammatory drug (NSAID) is an aromatic compound having two or more benzene rings.

6. The skin permeation enhancer according to claim 1, wherein the bioactive substance is at least one selected from the group consisting of mefenamic acid or a salt thereof, flavone, and hesperetin.

7. A skin permeation enhancement method for a bioactive substance, the method comprising a step of topically administering α-glucosylnaringin and a bioactive substance to the skin.
